(19) Europäisches Patentamt
European Patent Office
Office européen des brevets

(11) **EP 4 603 140 A1**

(12) **EUROPEAN PATENT APPLICATION**

(43) Date of publication:
**20.08.2025 Bulletin 2025/34**

(21) Application number: **25157143.6**

(22) Date of filing: **11.02.2025**

(51) International Patent Classification (IPC):
***A61N 5/10*** *(2006.01)*

(52) Cooperative Patent Classification (CPC):
**A61N 5/103**; A61N 2005/1034; A61N 2005/1087

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.02.2024 US 202418440739**

(71) Applicant: **Siemens Healthineers International AG
6312 Steinhausen (CH)**

(72) Inventors:
• **ROSSI, Michiko**
 **02700 Kaunianen (FI)**
• **FOLKERTS, Michael**
 **Costa Mesa, 92626 (US)**
• **LANSONNEUR, Pierre**
 **69004 Lyon (FR)**

(74) Representative: **Mathisen & Macara LLP
Charta House
30-38 Church Street
Staines-upon-Thames TW18 4EP (GB)**

(54) **RADIATION DOSE VOLUME AND RADIATION DOSE RATE VOLUME CALCULATION APPARATUS AND METHOD**

(57) A control circuit 101 accesses 201, 203 information regarding radiation delivery parameters 202 and a calculation algorithm 204 and calculates 205 both radiation dose and radiation dose rate volumes using the calculation algorithm and the information regarding radiation delivery parameters to provide at least one calculated radiation dose volume and at least one calculated radiation dose rate volume.

FIG. 2

## Description

Technical Field

These teachings relate to optimization of an energy-based treatment plan. A patient's planning target volume may be treated with energy pursuant to the energy-based treatment plan.

Background

The use of energy to treat medical conditions comprises a known area of prior art endeavor. For example, radiation therapy comprises an important component of many treatment plans for reducing or eliminating unwanted tumors. Unfortunately, applied energy (such as photons or protons) does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

It can be important to assess the quality of an optimized radiation treatment plan before administering radiation in accordance with that plan. Such an assessment may make use of dose volume and dose rate volume calculations. Unfortunately, calculating such values can be computationally expensive and hence either necessitate the availability of expensive computational platforms and/or require considerable time to make the calculations.

Summary

In one aspect, the present invention provides a method as defined in claim 1. In another aspect, the present invention provides apparatus as defined in claim 6. Optional features are specified in the dependent claims.

Brief Description of the Drawings

The above needs are at least partially met through provision of the radiation dose volume and radiation dose rate volume calculation apparatus and method described in the following detailed description, particularly when studied in conjunction with the drawings, wherein:

FIG. 1 comprises a block diagram as configured in accordance with various embodiments of these teachings;
FIG. 2 comprises a flow diagram as configured in accordance with various embodiments of these teachings;
FIG. 3 comprises a schematic representation as configured in accordance with various embodiments of these teachings;
FIG. 4 comprises a table as configured in accordance with various embodiments of these teachings;
FIG. 5 comprises a graph as configured in accordance with various embodiments of these teachings;
FIG. 6 comprises a schematic representation as configured in accordance with various embodiments of these teachings; and
FIG. 7 comprises a graph as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

Detailed Description

Generally speaking, pursuant to these various embodiments a control circuit accesses information regarding radiation delivery parameters and a calculation algorithm and calculates both radiation dose and radiation dose rate volumes using the calculation algorithm and the information regarding radiation delivery parameters to provide at least one calculated radiation dose volume and at least one calculated radiation dose rate volume.

The aforementioned radiation delivery parameters can include, for example, radiation beam delivery parameters such as, but not limited to, proton beam delivery parameters. (including position information for particles such as initial position information), velocity information for particles, energy, directional information for the particles, and/or temporal information corresponding to the particles.

The aforementioned calculation algorithm may comprise, by one approach, a probabilistic computation (such as, but not limited to, a Monte Carlo simulation), and/or, may include radiation beam delivery logic.

The calculated radiation dose volume and at least one calculated radiation dose rate volume can be employed, for example, when optimizing a radiation treatment plan for a particular patient (in particular, by providing a useful basis for evaluating the quality of an optimized plan), which optimized plan can then be utilized to administer therapeutic radiation to that patient.

So configured, and by one approach, these teachings will accommodate fast dose volume dose rate volume calculations by integrating beam delivery dynamics into Monte Carlo particle transport in radiation therapy treatment planning. The present teachings can be especially useful when both the dose volume and the dose rate volume are needed to evaluate the quality of an optimized radiation treatment plan because these teachings provide for reducing the computational cost of state-of-the-art calculations by performing both the dose volume and dose rate volume calculations simultaneously. By allowing a central processing unit or graphics processing unit to do similar operations in a row and by better cache usage, the dose and dose rate calculation can be significantly sped up.

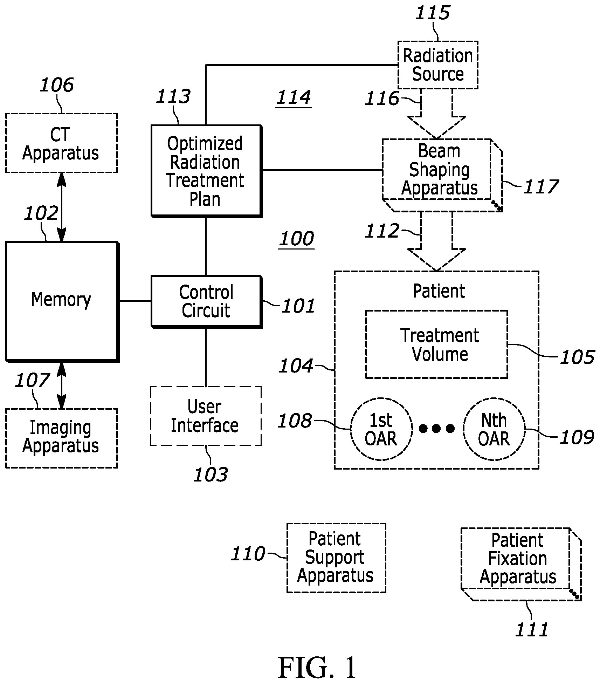

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101).

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons. These teachings will also support proton beam radiotherapy, in which case, the radiation source 115 may comprise a cyclotron or synchrotron that accelerates protons to high speeds. Those protons can then be directed through a series of magnets that shape and focus the proton beam.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

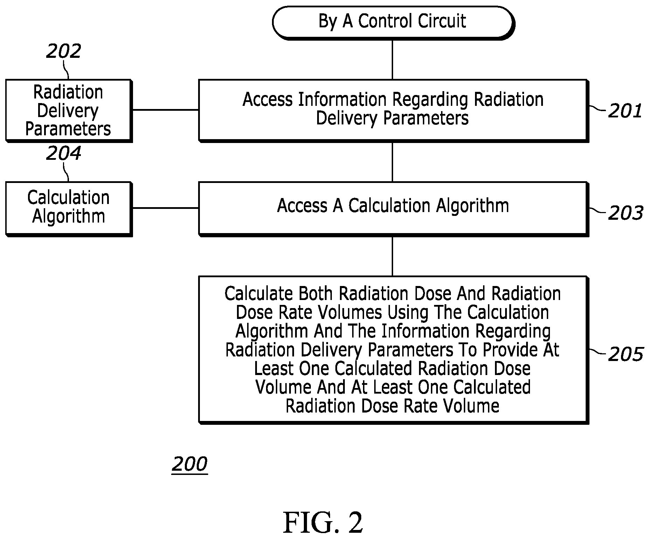

Referring now to FIG. 2, a process 200 that can be carried out, for example, in conjunction with the above-described application setting (and more particularly via the aforementioned control circuit 101) will be described. Generally speaking, this process 200 serves to facilitate providing an optimized radiation treatment plan 113 (such as a proton beam radiation treatment plan) to thereby facilitate treating a particular patient with therapeutic radiation using a particular radiation treatment platform per that optimized radiation treatment plan 113.

For the sake of an illustrative example, it is presumed in the description below that the radiation treatment at issue comprises a proton-based radiation treatment. Proton beam radiotherapy is a form of radiation therapy that utilizes protons, which are positively charged particles, to deliver high-energy radiation to cancer cells. Unlike traditional radiation therapy that uses X-rays or photons, proton beam therapy allows for more precise targeting of tumors while minimizing damage to surrounding healthy tissues. This is at least partly because protons have a unique property called the Bragg peak, which allows the protons to deposit most of their energy at a specific depth within the body.

It should also be noted that the foregoing proton beam radiotherapy may comprise so-called FLASH radiotherapy. FLASH energy treatment is a known area of prior art endeavor, albeit a relatively new area of consideration. FLASH radiotherapy is a technique (at least for photon and proton treatments) that employs very brief, very high dose rates (utilizing large beam currents). FLASH treatments hold the promise of shortening treatment time to just one to three 1-second sessions while also potentially reducing side effects, perhaps considerably. For example, a significant sparing of normal tissue notwithstanding iso-effective tumor growth delay has been demonstrated through very brief irradiation at dose rates on the order of 40 Gy's. This sparing of normal tissue has been dubbed the FLASH effect.

At block 201, this process 200 accesses information regarding radiation delivery parameters 202 (by accessing, for example, the aforementioned memory 102). These teachings will accommodate a variety of different radiation delivery parameters, including, but not limited to, proton beam delivery parameters (including position information for particles such as initial position information), velocity information for particles, energy and directional information for the particles, and/or temporal information corresponding to the particles (where the temporal information may comprise, for example, temporal information corresponding to dose accumulation in each voxel such as, by way of non-limiting examples, volume(s) related to a first instance of time when dose is received in each voxel and/or volume(s) information related to a final instance of time when a total dose is received in each voxel). Any one or more of the foregoing, for example, can comprise the accessed information.

At block 203, the control circuit 101 accesses a calculation algorithm 204 (again, by one approach, by accessing the aforementioned memory 102). This accessed calculation algorithm may comprise, by one approach, a probabilistic computation (such as, but not limited to, a Monte Carlo simulation), or, by another approach, may include radiation beam delivery logic. (Further description in these regards appears below.)

At block 205, the control circuit 101 calculates both radiation dose and radiation dose rate volumes using the accessed calculation algorithm 204 and the information regarding radiation delivery parameters 202 to provide at least one calculated radiation dose volume and at least one calculated radiation dose rate volume.

Further details that comport with these teachings will now be presented. It will be understood that the specific details of these examples are intended to serve an illustrative purpose and are not intended to suggest any particular limitations with respect to these teachings.

In modulated scanning proton FLASH radiotherapy, the dose rate used is often calculated, in part, by employing an influence matrix. The latter is typically generated by calculating the dose contribution of each spot and storing the results as an array of dose volumes. Deterministic or stochastic calculation models can be used for this. The dose rate for each voxel can then be calculated using that influence matrix. Calculating an influence matrix, however, can be computationally expensive in terms of both memory and time usage because the dose distribution is isolated for each spot. (As used herein, a "spot" refers to a location along the path of a proton beam where energy is deposited.) The present teachings can obviate calculating an influence matrix. These teachings can also combine the dose and dose rate calculation steps into a single step.

As one illustrative example, the dose and dose rate calculation task sequence can be as follows.

1. The beam line is calculated and/or optimized. The spots that cover the target are populated and/or optimized for plan dose quality.
2. A treatment plan is optimized, with the spots being modulated to optimize the dose.
3. (Optional) The dose rate is optimized. This step can be integrated in step 2 when the plan is optimized. The dose rate can be optimized, for example, by reorganizing the spots or adjusting the monitor unit rates and/or spot positions.
4. Postprocessing. The raw spot list can be analyzed and filtered to ensure that the treatment delivery is able to deliver the spots. A final spot list is created.
5. The dose and dose rate are calculated with a Monte Carlo particle transport calculation that includes:

    a. A beam delivery dynamics calculation;
    b. Particle generation; and
    c. The dose and dose rate calculation.

In the following example, a first step in a Monte Carlo-based dose calculation is particle generation where a very large number of random particles are sampled whose initial positions and initial velocity distributions match the radiation source being modeled. The batch of primary particles is then propagated in the medium in a stepwise fashion where the particles experience interactions with the medium deflecting them and slowing them down. As the particles slow down, they deposit energy (i.e., dose) to the medium. Primary particles can also kick off further secondary particles to be tracked.

In this example, the particle generation step employs the aforementioned radiation delivery parameters 202 regarding the machine delivery dynamics. This can mean that in addition to initial particle position and velocity information, a particle can have an associated time at which that particle is released from the source. In these regards, by integrating the treatment delivery system's beam delivery dynamics, the time at which a given particle is released from the source can be obtained and associated with that particle.

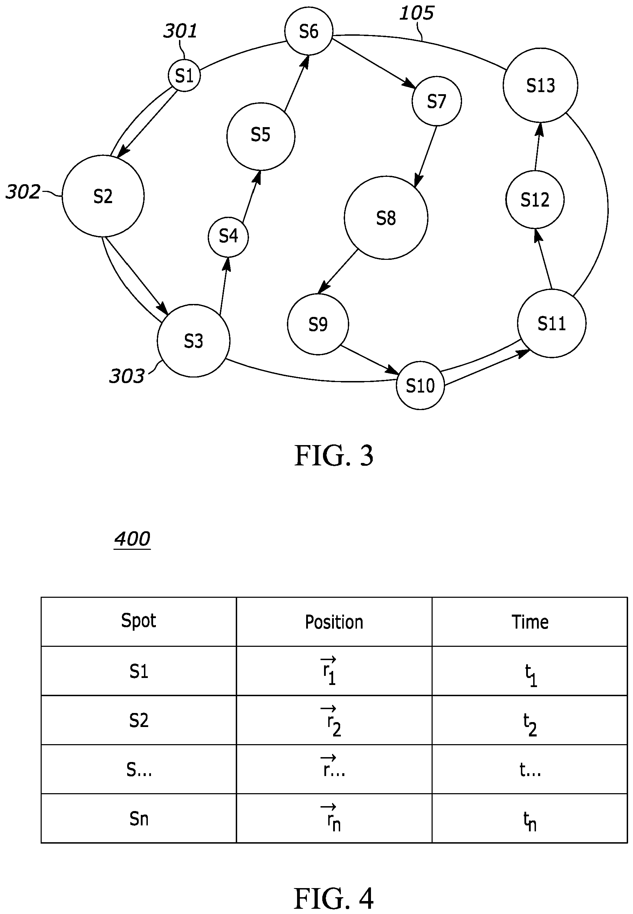

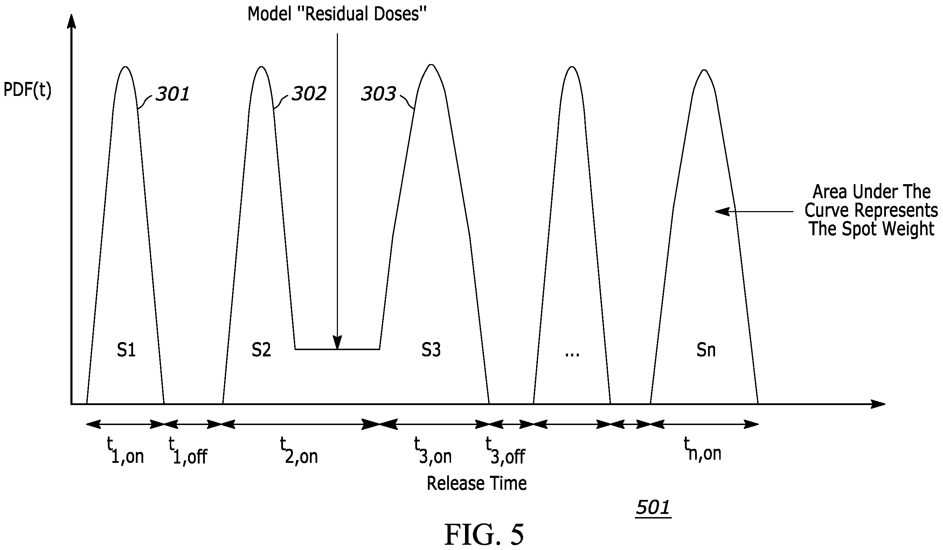

FIG. 3 presents a schematic depiction of a distribution of a plurality of spots (denoted S1 through S13 in this illustrative example) and their order of delivery within a patient's treatment volume 105. By calculating the aforementioned beam delivery dynamics, the instances of time when the beam is positioned at a certain location can be determined (as presented in this illustrative example of a corresponding table 400 presented in FIG. 4). The latter, in turn, supports generating a positional and temporal probability distribution function (PDF) for the particle generation (with FIG. 5 presenting an illustrative example in those regards).

A positional and temporal PDF 501 can model the different ways that spots are delivered. For example, the beam can be kept on only at a certain spot (say spot S1 301 in FIGS. 3 and 5) and the beam can also be kept on in between two spots (such as spots S2 302 and S3 303 in FIGS. 3 and 5).

By one approach as regards a Monte Carlo dose calculation, only the initial particle positions and velocities or initial positions, energy, and directions are the core information needed. For the Monte Carlo dose rate calculation, the additional information needed for the particle is the associated time at which the particle was released.

Large number of particles can be generated for each batch calculation. By one approach, the generated particles are sorted by their corresponding times of release. Each particle is then propagated in the medium. The first instant of time and the last instant of time are stored at which energy is deposited in a given position in the medium (or, by one approach, in a voxel) that represents the position in the medium. Those instances of time can then be associated to each particle's time of release. It may be noted that, in a typical application setting, the timescales of the particle transport in the medium will be significantly smaller and incomparable to the timescales in the beam delivery dynamics.

By one approach, a source particles array contains information about the initial position, direction, energy, and weight of each particle and the time in which each particle is released, thus allowing the dose rate to be simultaneously calculated with the dose.

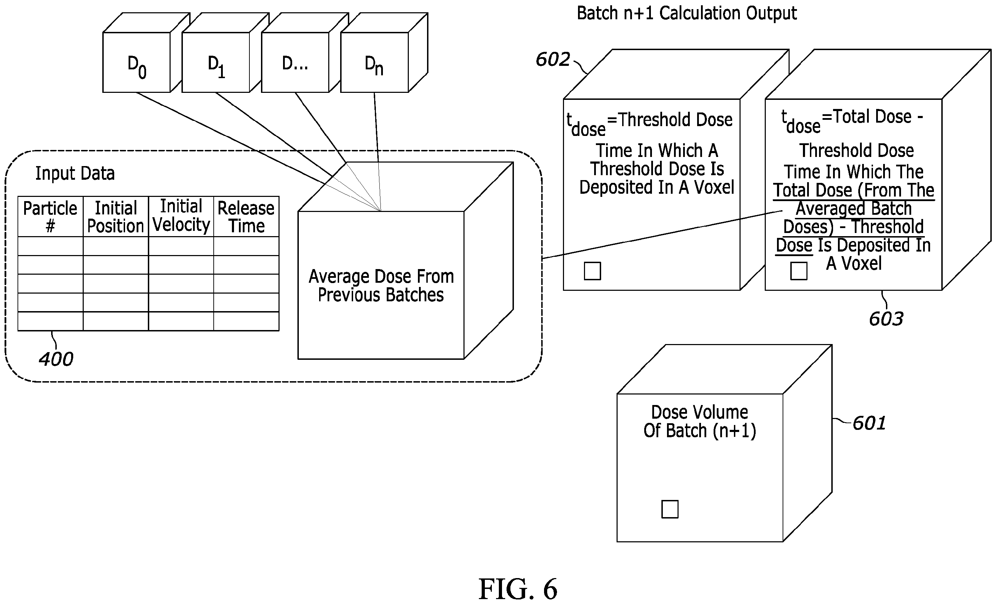

By one approach, three volume information components to be used for the Monte Carlo dose rate calculation are the dose volume, a first time volume that records the instance of time in which the voxel receives the threshold dose, and a second time volume that records the instance of time in which the voxel receives the total dose (from a previous calculation batch) minus the threshold dose. These three volumes are denoted in FIG. 6 by reference numerals 601-603, respectively.

In this example, the dose $D_i$ in every voxel is first calculated by summing the influence matrix over the spot indices:

$$D_i = \sum_{j=1}^{n} (d_{ij} MU_j),$$

where i refer to the voxel indices, j refer to the spot indices, $d_{ij}$ is the influence matrix [Gy/MU] (i.e., the dose from spot j in voxel i), and $MU_j$ is the monitor units of spot j [MU].

A pencil beam scanning dose rate can then be calculated for each voxel as:

$$\dot{D}_i = \begin{cases} 0, & \text{if } D_i \leq 3 \cdot d^t \\ \dfrac{D_i^*}{t_i^*}, & \text{if } D_i > 3 \cdot d^t \end{cases}$$

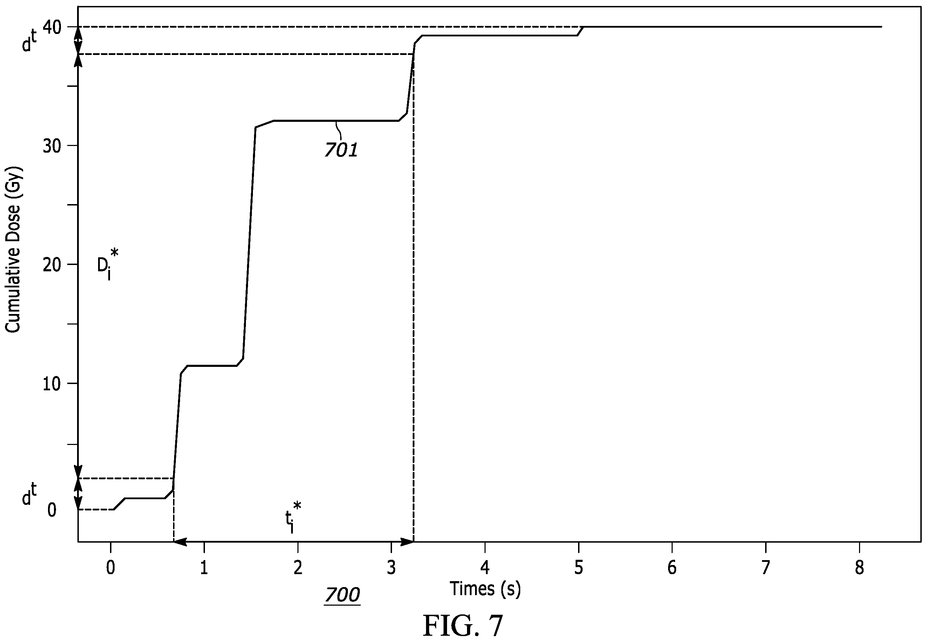

where $D_i^* = D_i - 2 \cdot d^t \, [\text{Gy}]$, $d^t$ is an arbitrary dose threshold [Gy] defined in the calculation options (a default value could be, for example, 0.01 Gy), and $t_i^*$ is the time needed for the accumulated dose to rise from $d^t$ to $D_i$ - $d^t$ [s], interpolated linearly as depicted in FIG. 7 (which presents a graph 700 that comprises an illustrative example of dose 701 accumulated in a single voxel as a function of time, where the dose rate is calculated as $D_i^*/t_i^*$). This information comprises the time difference of the last and first instance of time recorded in the time volume information component.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method comprising:
   by a control circuit:

   accessing information regarding radiation delivery parameters;
   accessing a calculation algorithm; and
   calculating both radiation dose and radiation dose rate volumes using the calculation algorithm and the information regarding radiation delivery parameters to provide at least one calculated radiation dose volume and at least one calculated radiation dose rate volume.

2. The method of claim 1 wherein the radiation delivery parameters include radiation beam delivery parameters.

3. The method of claim 2 wherein the radiation beam delivery parameters comprise proton beam delivery parameters.

4. The method of claim 3 wherein the proton beam delivery parameters include position information for particles.

5. The method of claim 1, 2, 3 or 4 wherein the calculation algorithm includes radiation beam delivery logic.

6. An apparatus comprising:

   a memory having information regarding radiation delivery parameters and a calculation algorithm stored therein;
   a control circuit operably coupled to the memory and configured to:

   - access the memory to access the information regarding the radiation delivery parameters;
   - access the calculation algorithm; and

- calculate both radiation dose and radiation dose rate volumes using the calculation algorithm and the information regarding radiation delivery parameters to provide at least one calculated radiation dose volume and at least one calculated radiation dose rate volume.

7. The apparatus of claim 6 wherein the radiation delivery parameters include radiation beam delivery parameters.

8. The apparatus of claim 7 wherein the radiation beam delivery parameters comprise proton beam delivery parameters.

9. The apparatus of claim 8 wherein the proton beam delivery parameters include position information for particles.

10. The method of claim 4 or the apparatus of claim 9 wherein the position information for the particles includes initial position information.

11. The method of claim 4 or the apparatus of claim 9 wherein the proton beam delivery parameters include:

velocity information for the particles; and/or energy and directional information for the particles.

12. The method of claim 4 or the apparatus of claim 9 wherein the proton beam delivery parameters include temporal information corresponding to the particles.

13. The method or apparatus of any one of claims 1 to 12 wherein the calculation algorithm comprises a probabilistic computation.

14. The method or apparatus of claim 13 wherein the probabilistic computation comprises a probabilistic computation of temporal information of beam particles as a function, at least in part of beam delivery dynamics and parameters.

15. The method or apparatus of claim 13 wherein the calculation algorithm comprises a Monte Carlo simulation.

*115*

Radiation
Source

*106*

CT
Apparatus

*113*  *114*  *116*

Optimized
Radiation
Treatment
Plan

Beam
Shaping
Apparatus

*117*

*102*

*100*  *112*

Memory

Control
Circuit

*101*

Patient

Treatment
Volume

*105*

*104*

*107*

*108*  1st
OAR

Nth
OAR

*109*

Imaging
Apparatus

User
Interface

*103*

*110*  Patient
Support
Apparatus

Patient
Fixation
Apparatus

*111*

FIG. 1

By A Control Circuit

*202*

Radiation Delivery Parameters

Access Information Regarding Radiation Delivery Parameters ~*201*

*204*

Calculation Algorithm

Access A Calculation Algorithm ~*203*

Calculate Both Radiation Dose And Radiation Dose Rate Volumes Using The Calculation Algorithm And The Information Regarding Radiation Delivery Parameters To Provide At Least One Calculated Radiation Dose Volume And At Least One Calculated Radiation Dose Rate Volume ~*205*

*200*

FIG. 2

FIG. 3

400

| Spot | Position | Time |
|------|----------|------|
| S1 | $\vec{r}_1$ | $t_1$ |
| S2 | $\vec{r}_2$ | $t_2$ |
| S... | $\vec{r}_{...}$ | $t_{...}$ |
| Sn | $\vec{r}_n$ | $t_n$ |

FIG. 4

Model "Residual Doses"

PDF(t)

*301*  *302*  303

Area Under The Curve Represents The Spot Weight

S1  S2  S3  ...  Sn

$t_{1,on}$  $t_{1,off}$  $t_{2,on}$  $t_{3,on}$  $t_{3,off}$  $t_{n,on}$

Release Time

*501*

FIG. 5

FIG. 6

EP 4 603 140 A1

FIG. 7

Europäisches
Patentamt
European
Patent Office
Office européen
des brevets

# EUROPEAN SEARCH REPORT

**Application Number**

EP 25 15 7143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/016108 A1 (KHUNTIA DEEPAK [US] ET AL) 21 January 2021 (2021-01-21) * paragraphs [0009] - [0015], [0045] - [0109]; figures 1-5,8-11 * | 1-15 | INV. A61N5/10 |
| X | US 2023/001233 A1 (PFEILER TINA [DE] ET AL) 5 January 2023 (2023-01-05) * the whole document * * paragraphs [0009], [0090], [0145] * | 1-15 | |
| X | US 2022/323791 A1 (KANG MINGLEI [US] ET AL) 13 October 2022 (2022-10-13) * the whole document * * paragraphs [0595], [0060], [0077] - [0079], [0115] - [0145] * | 1-15 | |
| X | US 2021/393982 A1 (LANSONNEUR PIERRE [FI] ET AL) 23 December 2021 (2021-12-23) * the whole document * * paragraph [0069] * | 1-15 | |
| A | US 2023/191150 A1 (HIRVONEN PETRI [FI] ET AL) 22 June 2023 (2023-06-22) * paragraphs [0004] - [0019], [0031] - [0034]; figures 1-3 * | 1-15 | **TECHNICAL FIELDS SEARCHED (IPC)** A61N |
| A | US 2023/390579 A1 (LIU WEI [US] ET AL) 7 December 2023 (2023-12-07) * the whole document * | 1-15 | |
| A | US 2022/118282 A1 (SOUKUP MARTIN [AT] ET AL) 21 April 2022 (2022-04-21) * the whole document * * paragraph [0063] * | 1-15 | |

The present search report has been drawn up for all claims

| Place of search | Date of completion of the search | Examiner |
|---|---|---|
| Munich | 28 May 2025 | Rodríguez Cosío, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 15 7143

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2025

| Patent document cited in search report | Publication date | Patent family member(s) | | Publication date |
|---|---|---|---|---|
| US 2021016108 A1 | 21-01-2021 | CN | 113543845 A | 22-10-2021 |
| | | EP | 3934750 A1 | 12-01-2022 |
| | | US | 2020282232 A1 | 10-09-2020 |
| | | US | 2021016108 A1 | 21-01-2021 |
| | | US | 2023125147 A1 | 27-04-2023 |
| | | WO | 2020181118 A1 | 10-09-2020 |
| US 2023001233 A1 | 05-01-2023 | CN | 117651586 A | 05-03-2024 |
| | | EP | 4363055 A1 | 08-05-2024 |
| | | US | 2023001233 A1 | 05-01-2023 |
| | | WO | 2023275027 A1 | 05-01-2023 |
| US 2022323791 A1 | 13-10-2022 | EP | 4323061 A1 | 21-02-2024 |
| | | JP | 2024513987 A | 27-03-2024 |
| | | US | 2022323791 A1 | 13-10-2022 |
| | | WO | 2022221412 A1 | 20-10-2022 |
| US 2021393982 A1 | 23-12-2021 | NONE | | |
| US 2023191150 A1 | 22-06-2023 | CN | 116271566 A | 23-06-2023 |
| | | EP | 4197594 A1 | 21-06-2023 |
| | | US | 2023191150 A1 | 22-06-2023 |
| US 2023390579 A1 | 07-12-2023 | NONE | | |
| US 2022118282 A1 | 21-04-2022 | CN | 116457061 A | 18-07-2023 |
| | | EP | 4228752 A1 | 23-08-2023 |
| | | US | 2022118282 A1 | 21-04-2022 |
| | | WO | 2022082127 A1 | 21-04-2022 |
| | | WO | 2022082128 A1 | 21-04-2022 |

EPO FORM P0459

For more details about this annex : see Official Journal of the European Patent Office, No. 12/82